# EUROPEAN PATENT APPLICATION

(11) **EP 1 374 803 A1**
(43) Date of publication of application: **02.01.2004**
(21) Application number: 03253909.0
(22) Date of filing: 20.06.2003
(51) Int. Cl.: A61F 2/30

(54) **Hip joint having a variety of trochanteric flange sizes**

(30) Priority: 21.06.2002 US 177720
(71) Applicant: Biomet, Inc., Warsaw, IN 46580 (US)
(72) Inventor: Brown, David R., Warsaw, Indiana 46580 (US); Les, Kimberly A., Troy, Michigan 48085 (US)
(74) Representative: Giles, Ashley Simon

(57) **Abstract**

The present invention provides a hip prosthesis (38) with a modular or integral trochanteric flange (56). The prosthesis (38) includes a stem (42) for insertion within a reamed out femoral canal and a head (50) for articulation within an acetabulum of a hip bone, the head (50) and stem (42) being connected by a neck (44). The neck (44) contains an attachment region (54) for securing the trochanteric flange (56) to the prosthesis (38). The trochanteric flange (56) is selected from a variety of trochanteric flanges differing in length. The specific trochanteric flange (56) selected depends upon the amount of hip joint soft tissue resected from a particular patient. If a large amount of soft tissue is resected an elongated trochanteric flange (56) will be selected. However, if little or no soft tissue is resected, a shorter trochanteric flange (56) will be selected. The ability to customize the size of the trochanteric flange (56) allows the physician to better recreate the natural relationship between the greater trochanter and the soft tissue of the hip joint, thus allowing the soft tissue to be secured to the trochanteric flange (56) without having to stretch the soft tissue or otherwise cause undesirable tension within the soft tissue.

## Description

### FIELD OF THE INVENTION

The present invention generally relates to implants for use in bone surgery. More specifically, the present invention relates to a hip implant for replacement of the proximal portion of the femur, the implant having a variety of flange sizes for greater trochanter replacement.

### BACKGROUND OF THE INVENTION

Hip joint replacement and reconstruction is common in modern medicine and is often required due to injury or the presence of cancerous cells in the bones comprising the hip joint and/or in the surrounding soft tissue. For example, resection of the proximal portion of the femur and the surrounding soft tissue is often required due to the presence of cancerous cells. Once the infected region of the proximal femur is resected, it is replaced with a prosthetic device that is shaped to correspond to the shape of the proximal portion. The prosthetic device is similar to the natural proximal femur in that it includes, in part, a stem, a neck, a head, an acetabular cup. However, current hip prosthesis appear to ignore the greater trochanter area of the natural hip joint.

To secure the prosthetic device to the remaining portion of the femur, the intramedullary canal of the femur is reamed and the stem portion of the prosthetic device is inserted within the reamed-out portion of the intramedullary canal. The prosthesis is secured within the reamed-out portion using a suitable device or method such as impaction of the hip prosthesis or bone cement.

In its natural state, the greater trochanter closely approximates the soft tissue surrounding the hip joint and has surfaces for attachment to the soft tissue. The close proximity of the greater trochanter to the surrounding soft tissue is desirable as it allows the greater trochanter to be secured to the soft tissue without having to stretch or strain the soft tissue to reach the greater trochanter. However, the proximal relationship between the greater trochanter and the surrounding soft tissue is difficult to recreate using current femoral implants.

Recreation of the proximal relationship between the greater trochanter and the surrounding soft tissue using current femoral implants is difficult because current implants provide for the use of only a single integral trochanteric flange area of a fixed length and size that does not readily have a soft tissue attachment region. The result of using a femoral implant with an integral fixed length trochanteric flange is that the distance between the trochanteric flange and the surrounding soft tissue will vary according to the amount of soft tissue resected. If a large amount of soft tissue is resected, the distance between the trochanteric flange and the soft tissue to be attached to the flange is great. Consequently, as the trochanteric flange is secured to the soft tissue using, for example, wire sutures, the tension exerted on the wire sutures will often cause the sutures to break, leaving the trochanteric flange detached from the surrounding soft tissue. In other instances, the distance between the trochanteric flange and the remaining soft tissue will be so great that attachment of the soft tissue to the trochanteric flange is not possible. Conversely, if resection of the soft tissue is not necessary or if only a small portion requires resection, the trochanteric flange will be too long and will cause undesirable pressure upon the soft tissue unless the flange is cut down. In all of the above situations, the end result is that the patient experiences an undesirable loss of hip joint motion.

In order to recreate the natural relationship between the trochanteric flange and the surrounding soft tissue, there is a need for a femoral implant capable of having trochanteric flanges of differing sizes according to the amount of soft tissue resected. For example, if a large amount of soft tissue is resected a large trochanteric flange will be selected for use with the femoral implant so as to provide for close approximation between the flange and the soft tissue. Conversely, if little or no soft tissue is resected then a smaller trochanteric flange will be selected. Re-creation of the natural relationship between the greater trochanter and the surrounding soft tissue using a femoral implant having trochanteric flanges of different sizes allows the greater trochanter to be easily secured to the surrounding soft tissue without undesirably stretching the soft tissue or cutting down the trochanteric flange. Moreover, by providing a modular trochanteric flange inventory tracking may be reduced and interoperative surgical options increased. Furthermore, by providing a trochanteric flange with an attachment region, attachment of soft tissue to the trochanter flange is facilitated.

### SUMMARY OF THE INVENTION

The present invention overcomes the prior art deficiencies by providing a hip prosthesis with trochanteric flanges of different sizes. The prosthesis includes a stem for insertion within a reamed out intramedullary canal and a head operable to receive an acetabular cup for articulation within an acetabulum of a hip bone. The head and stem are connected by a neck. The neck contains either an integral trochanteric flange having an attachment region selected from a variety of sizes or an attachment region for securing a modular trochanteric flange to the prosthesis. The trochanteric flange is selected from a variety of trochanteric flanges differing in length and shape. The specific trochanteric flange selected depends upon the amount of hip joint soft tissue resected from a particular patient. For example, if a large amount of soft tissue is resected then an elongated trochanteric flange will be selected. However, if little or no soft tissue is resected, a shorter trochanteric flange will be selected. The ability to customize the size of the trochanteric flange allows the physician to better recreate the natural relationship between the greater trochanter and the soft tissue of the hip joint, thus allowing the soft tissue to be secured to the trochanteric flange without having to stretch the soft tissue or otherwise cause undesirable tension within the soft tissue.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### BRIEF DESCRIPTION OF THE DRAWINGS

The present invention will become more fully understood from the detailed description and the accompanying drawings, wherein:

Figure 1 is an anterior view of the bones and associated soft tissue of the right human hip joint;

Figure 2 is a perspective view of the prosthesis of the present invention outfitted with a modular trochanteric flange and a femur with a resected proximal portion;

Figure 3 is a perspective view of the trochanteric flange of Figure 2, the soft tissue attachment region having through holes positioned in a first orientation;

Figure 3a is a perspective view of the trochanteric flange of Figure 2, the soft tissue attachment region having through holes positioned in a second orientation;

Figure 4 is a side view of the trochanteric flange of Figure 2 secured to the prosthesis of Figure 2;

Figure 4a is a cross-section of Figure 4 taken along line 4a;

Figure 4b is a sectional view of the prosthesis of the current invention with a modular trochanteric flange secured to the prosthesis by way of a taper fit;

Figure 4c is a perspective view of the prosthesis of the current invention with a modular trochanteric flange secured to the prosthesis by way of a lateral, dove-tail taper fit;

Figure 4d is a perspective view of the prosthesis of the current invention with a modular trochanteric flange secured to the prosthesis by way of a distal to proximal, dove-tail taper fit;

Figure 4e is a perspective view of the prosthesis of the current invention with a modular trochanteric flange secured to the prosthesis by way of a wedge-shaped taper fit;

Figure 4f is a perspective view of the prosthesis of the current invention with a modular trochanteric flange secured to the prosthesis by way of a dove-tailed, wedge-shaped taper fit;

Figure 4g is a sectional view of the prosthesis of the current invention with a modular trochanteric flange secured to the prosthesis by way of a fastening device;

Figure 4h is a sectional view of the prosthesis of the current invention with a modular trochanteric flange secured to the prosthesis in a snap-fit relationship;

Figure 5 is a sectional view of a natural greater trochanter secured to the prosthesis of the current invention by way of a fastening device;

Figure 6 is a side view of a trochanteric flange having an extended soft tissue attachment region;

Figure 7 is a side view of a trochanteric flange having a medium length soft tissue attachment region;

Figure 8 is a side view of a trochanteric flange having a shortened soft tissue attachment region;

Figure 9 is an anterior view of the hip joint of Figure 1, the proximal portion of the femur and a portion of the surrounding soft tissue having been resected and replaced with the hip prosthesis of Figure 2, the prosthesis is outfitted with the trochanteric flange of Figure 6; and

Figure 10 is an anterior view of the hip joint of Figure 1, the proximal portion of the femur having been resected and replaced with the hip prosthesis of Figure 2, the prosthesis is outfitted with the trochanteric flange of Figure 8.

Figure 12 is an exploded sectional view of the hip joint of Figure 1, the proximal portion of the femur having been resected and replaced with the hip prosthesis of Figure 2.

### DETAILED DESCRIPTION OF THE PREFERRED EMBODIMENTS

The following description of the preferred embodiment(s) is merely exemplary in nature and is in no way intended to limit the invention, its application, or uses. More specifically, even though a modular implant is illustrated, the implant of the present invention may be integral or contain any combination of modular and integral components including the trochanteric flange. The components may also be of various different sizes according to the particular application.

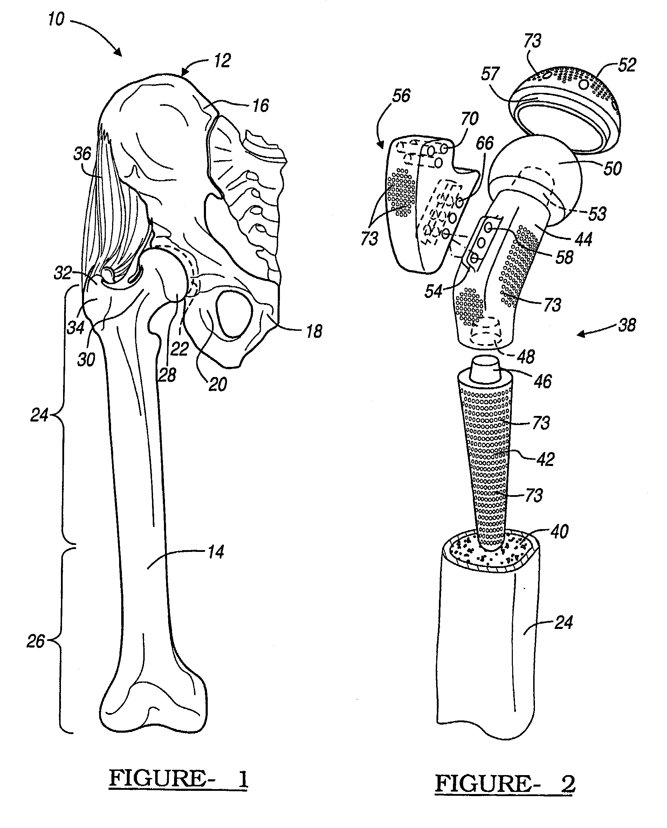

With reference to Figure 1, an anterior view of a right human hip joint 10 is illustrated. More specifically, hip bone 12 and femur 14 are illustrated. The hip bone 12 is comprised of an ilium 16, an ischium 18, and a pubis 20. Union of the ilium 16, ischium 18, and pubis 20 takes place in and around acetabulum 22, a large cup-shaped articular cavity.

The femur 14 has a proximal region 24 and a distal region 26. The proximal region 24 is comprised of a head 28, a neck 30, a greater trochanter 32, and a lesser trochanter 34. The greater trochanter 32 projects from the femur 14 and serves as a point of attachment to surrounding soft tissue 36. Head 28 is supported upon neck 30 and is received by and pivots within acetabulum 22, thus providing the hip joint 10 with ball-and-socket articulation.

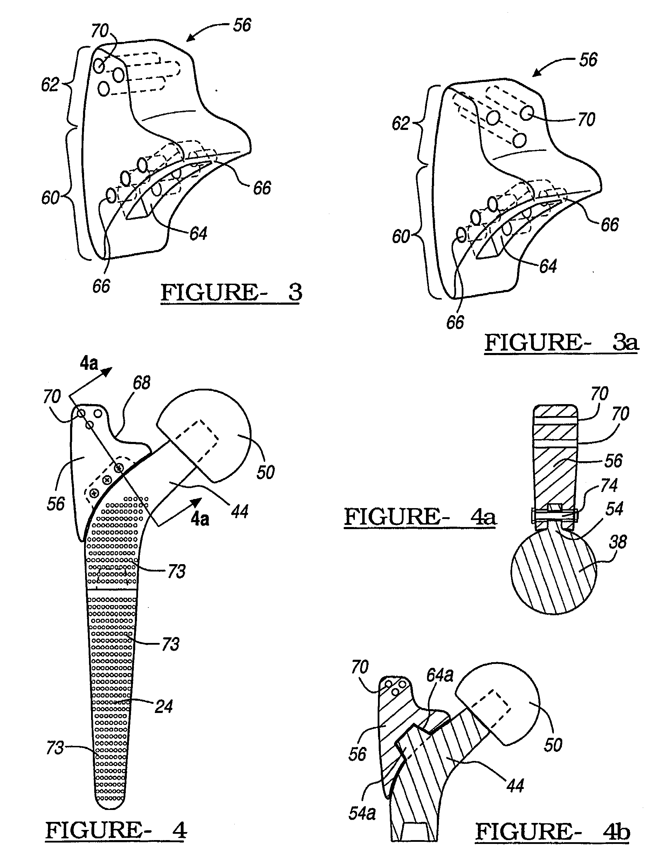

Due to injury or disease, such as the infiltration of cancerous cells, it may become necessary to resect portions of the femur 14 and portions of the soft tissue 36 surrounding the femur 14. In severe instances it may even become necessary to resect the entire proximal region 24 including the head 28, neck 30, and greater trochanter 32. In such circumstances, the resected portions of the proximal femur 24 may be replaced with a prosthesis 38 as shown in Figure 2.

Resection of the proximal portion 24 of femur 14 exposes intramedullary canal 40. The intramedullary canal 40 is reamed to receive a stem 42 of prosthesis 38. The stem 42 may be tapered (as illustrated in Figure 2), curved, or of any other suitable shape or size. The stem 42 is secured within the femur 14 using any suitable method such as impaction or any suitable material such as bone cement.

In addition to stem 42, the prosthesis 38 includes a neck 44 of any suitable size. The stem 42 may be integral with the neck 44 or modular. If the connection between the stem 42 and neck 44 is modular, the stem 42 may be secured to the neck 44 through the use of a variety of devices or methods such as a Morse taper fit. Figure 2 provides an example of a taper fit with male portion 46 of the taper extending from the stem 42 and female portion 48 formed within the neck 44. It must be noted that this configuration may be reversed so that the male portion 46 extends from the neck 44 and the female portion 48 is within the stem 42.

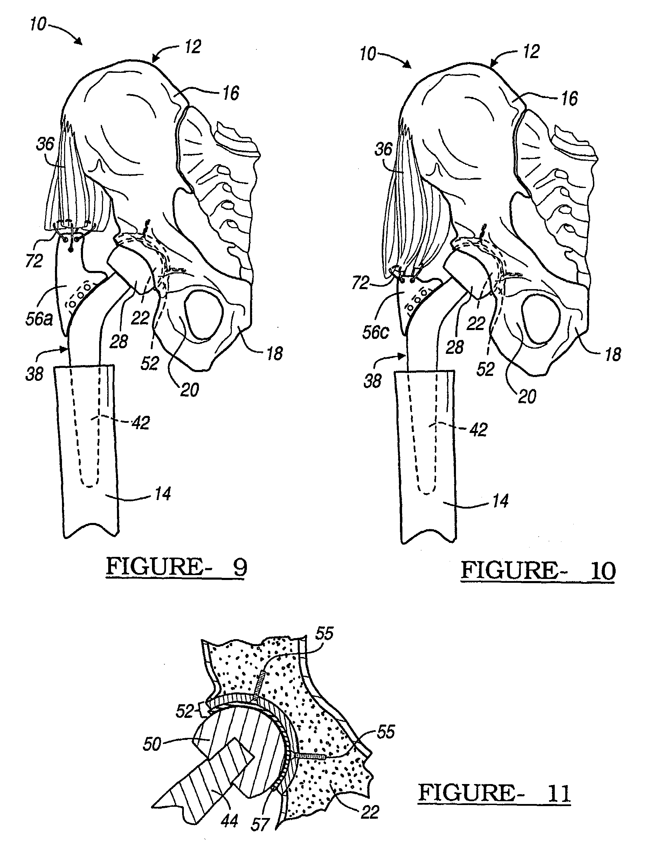

Disposed at the end of neck 44 opposite stem 42 is a suitably sized head 50. The head 50 is operable to articulate with a prosthetic acetabular cup 52 (Figure 11) or natural acetabulum 22. The head 50 may either be integral with the neck 44 or modular, as seen in Figure 2. If the head 50 is modular, it may be attached to the neck 44 by way of a Morse taper 53 or any other appropriate connection device or mechanism. The acetabular cup 52 may be of various different sizes and shapes so as to be properly received by the acetabulum 22 and articulate with properly sized head 50. The acetabular cup 52 is attached to the acetabulum 22 using any known attachment technique, such as bone screws 55 (Figure 11). The acetabular cup 52 may be configured as a shell with a polymer insert or bearing liner 57(Figure 11), may be formed as a single shell having an inner articulating spherical bearing surface, or may be any other type of acetabular cup 52.

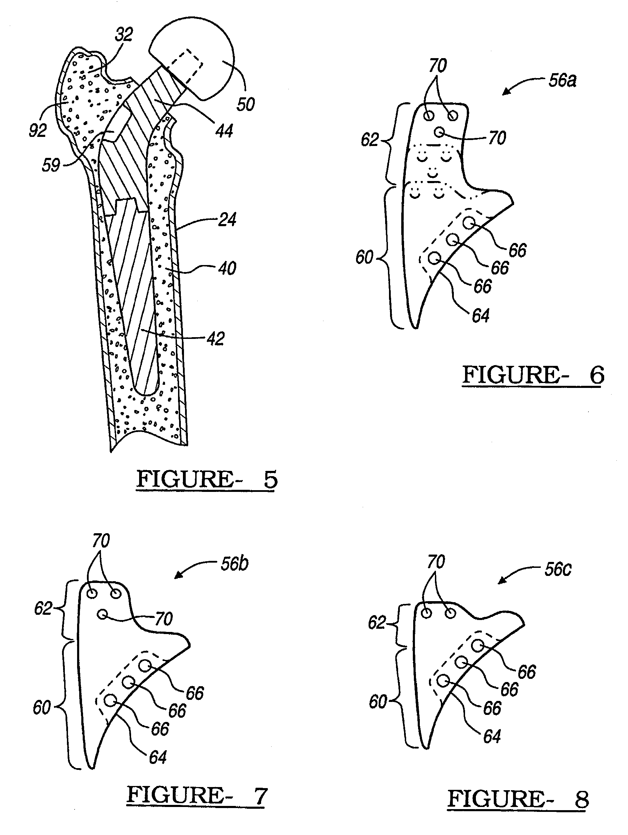

The neck 44 also includes trochanter attachment portion 54 for securing a trochanteric flange 56 to the neck 44. The trochanter attachment portion 54 is generally in the shape of a boss extending from the neck 44. The attachment portion 54 preferably comprises at least one through hole 58 for receiving a suitable fastening device such as a screw or bolt. However, the trochanter attachment portion 54 may be of any size or shape suitable for properly securing the trochanteric flange 56 to the neck 44. In this regard, the neck 44 may also be configured with a recess 59 (Figure 5) for use in attaching the trochanteric flange 56.

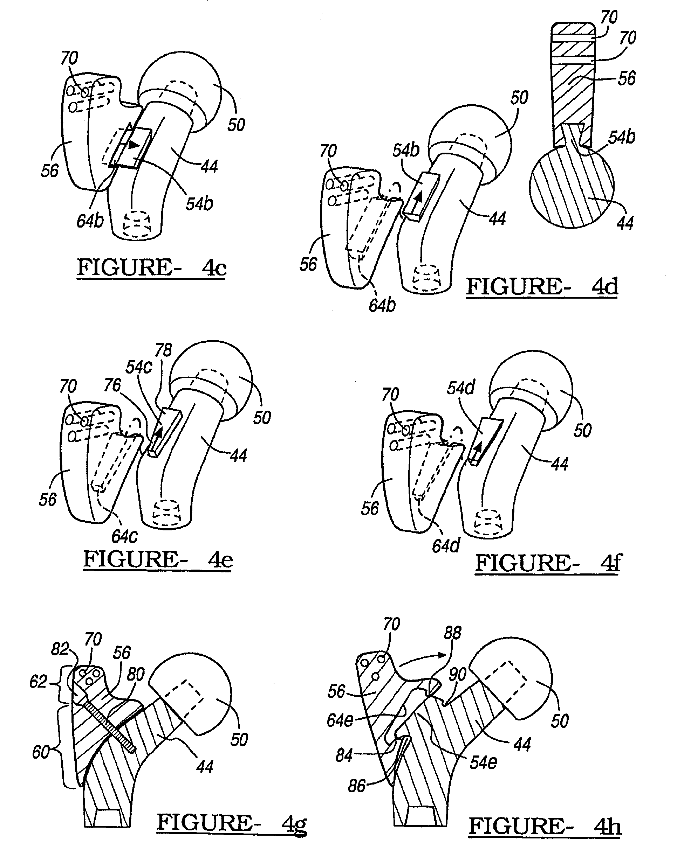

The trochanteric flange 56, as seen in Figures 3 and 4, comprises a prosthesis attachment region 60 and a soft tissue attachment region 62. The prosthesis attachment region 60 is a broad base region that abuts the neck 44 and is secured to the attachment portion 54 using any suitable device or method. As illustrated, the prosthesis attachment region 60 includes a recessed portion 64 capable of receiving and securing the trochanteric flange 56 to the attachment portion 54 of prosthesis 38. The recessed portion 64 preferably includes at least one through hole 66 for receiving a suitable fastening device. The recessed portion 64 nestingly receives the boss 54 of the neck 44. It must be noted that this configuration may also be reversed such that flange 56 includes a boss, similar to boss 54, capable of being received by recess 59.

The soft tissue attachment region 62 extends from the prosthesis attachment region 60 upward and away from the neck 44 towards the ilium 16. The soft tissue attachment region 62 is approximately half the width of the prosthesis attachment region 60, thus forming recess 68, as seen in Figure 4. The presence of recess 68 is advantageous as it allows head 50 to freely rotate and pivot without being restrained by trochanteric flange 56.

The soft tissue attachment region 62 includes at least one through hole 70 for receiving a suitable attachment device, such as sutures 72 (Figures 9 and 10), so as to secure the trochanteric flange 56 to the surrounding soft tissue 36. The through hole 70 may be of any suitable size or configuration and may be oriented to extend through the soft tissue attachment region 62 in any suitable direction, such as laterally (Figure 3) or medially (Figure 3a). Another possible orientation for through hole 70 is positioning through hole 70 such that it extends through soft tissue attachment region 62 in a crossing pattern, such as an "X" pattern. Further, through hole 70 may be positioned such that it extends longitudinally through the soft tissue attachment region 62 and prosthesis attachment region 60.

The stem 42, neck, 44, head 50, acetabular cup 52, and flange 56 are all made of a suitable biocompatible material and are preferably made of a metal such as titanium, stainless steel, titanium alloy, cobalt-chrome-molybdenum alloy, etc. Further, the outer surfaces of the stem 42, neck 44, head 50, acetabular cup 52, and flange 56 may be covered with a porous region or a porous coating 73. The porous regions 73 may be used to promote both soft or hard tissue ingrowth.

The trochanteric flange 56 may be secured to the neck 44 in numerous different ways. For example, the flange 56 may be seated atop the attachment portion 54, as seen in Figures 4 and 4a. It must be noted that this orientation may be reversed, as described above, such that a boss, similar to boss 54, extends from flange 56 for receipt within recess 59. Once aligned, a suitable fastening device, such as bolt 74 or a pin (not shown), may be inserted through the aligned holes 58 and 66 or, in the absence of holes 58 and 66, driven directly through the flange 56 and neck 44 to secure the flange 56 into position. Further, flange 56 may be secured to neck 44 using any suitable adhesive, such as cement. Still further, flange 56 may be secured to neck 44 through pinching action between boss 54 and recess 64 or by providing boss 54 and recess 64 with any other suitable configuration such that when boss 54 and recess 64 are combined a secure fit is established. Possible configurations include a dovetail configuration or a configuration having a suitable catch. Any combination of any of the above configurations, and any other suitable configuration, may also be used. It must be noted that while flange 56 is secured to neck 44 of prosthesis 38, the flange 56 may be secured to any other suitable prosthetic device.

The use of a recessed portion 64a and an attachment portion 54a made of a suitable metal permits the fastening of the flange 56 to the neck 44 by way of a Morse taper, as seen in Figure 4b. More specifically, as illustrated the recessed portion 64a acts as the female portion of the Morse taper while the modular trochanter attachment portion 54a acts as the male portion of the morse taper. Insertion of the recessed portion 64a over the attachment portion 54a completes the Morse taper and secures the trochanteric flange 56 to the prosthesis 38. Trochanter attachment portion 54a and recessed portion 64a are identical to trochanter attachment portion 54 and recessed portion 64 except that through holes 58 and 66 are absent.

In addition to the taper fit shown in Figure 4b, numerous other taper fit configurations may be used to secure the trochanteric flange 56 to the trochanter attachment portion 54. For example, a "dove-tailed" trochanter attachment portion 54b may be used (Figure 4c). The attachment portion 54b becomes wider as it extends from neck 44. To receive the "dove-tailed" attachment portion 54b, a flange 56 having a tapered recessed portion 64b is used. The recessed portion 64b is tapered such that it complements the trochanter attachment portion 54b. To secure the flange 56 to the neck 44, the tapered recessed portion 64b is laterally slid into engagement with the trochanter attachment portion 54b. To permit the lateral sliding of the flange 56 upon the attachment portion 54b, the portions of the recessed portion 64b that contain apertures 66 are removed. Further, apertures 58 are not present in trochanter attachment portion 54b. It must be noted that in addition to sliding the flange 56 upon the attachment portion 54b laterally, the flange 56 may also be slid upon the attachment portion 54b in a direction from the distal end of the attachment portion 54b to the proximal end, the end closest to the head 50. To permit such engagement, the recessed portion 64b must be open at one end as seen in Figure 4d.

A wedged shaped taper may also be used to secure the trochanteric flange 56 to the neck 44 as seen in Figure 4e. To effectuate the wedged taper an attachment portion 54c is used. The attachment portion 54c is shaped such that end 76, the end distal to the head 50, is the most narrow part of the attachment portion 54c and that end 78, the end proximal to the head 50, is the widest portion. The recessed portion 64c of the trochanteric flange 56 is of a shape complementary to the attachment portion 54c. As the trochanteric flange 56 is slid onto the attachment portion 54c, from the direction of the stem 44 to the head 50, the taper is completed and the flange 56 is secured to attachment portion 54c. Preferably, the wedge taper is a dove-tail taper having a trochanter attachment portion 54d that becomes wider as it extends from the neck 44 (Figure 4f). To receive trochanter attachment portion 54d, a recessed portion 64d that is dove-tailed is used, the recessed portion 64d being at its widest point furthest from the neck 44.

The trochanteric flange 56 may also be secured to the neck 44 using an elongated fastening device such as screw 80 (Figure 4g). The screw 80 extends through an aperture 82 of the trochanteric flange 56 to engage the neck portion 44. The aperture extends the length of the flange 56 from the soft tissue attachment region 62 to the prosthesis attachment region 60. The screw 80 may engage neck portion 44 by either being received by a suitable receptor or by being driven directly into the neck 44.

The trochanteric flange 56 may also interlock with attachment portion 54 as seen in Figure 4h. To carryout such an interlocking attachment, a recessed portion 64e having a tab 84 is used. The tab 84 is received by a recess 86 of the trochanter attachment portion 54e. Once the tab 84 is locked within the recess 86, the flange 56 is rocked such that a second tab 88 of the flange 56 is received and locked within a second recess 90 within trochanter attachment portion 54e. Thus, through the use of tabs 84, 88 and recesses 86, 90, the trochanteric flange 56 may be secured to the attachment portion 54e.

The trochanteric flange 56 may also be secured to the attachment portion 54 using a combination of the above attachment systems or any other suitable attachment system. For example, in addition to securing the trochanteric flange 56 to the attachment portion 54 using a taper fit, such as the taper fit of Figure 4c, a fastening device, such as screw 80 of Figure 4g, extending through the flange 56 to engage the neck 44 may also be used. The use of both a taper fit and a fastening device is advantageous as it enhances the attachment of the trochanteric flange 56 to the neck 44.

In addition to the above described attachment methods, it is contemplated that the trochanteric flange 56 may be secured to prosthesis 38 in any other suitable manner. Further, the prosthesis attachment region 60 and the trochanter attachment portion 54 may take the form of any shape or configuration in addition to those described above so as to effectuate the attachment of the trochanteric flange 56 to the prosthesis 38. For example, the configuration of attachment portion 54 and recessed portion 64 may be reversed with recess 59 being formed within neck 44 (Figure 5) and a corresponding flange extending from the prosthesis attachment region 60 (not shown). Additionally, the trochanteric flange 56 may be integral with the prosthesis 38.

The prosthesis 38 having the recess 59 formed within the neck 44 may be used in situations where total resection of the greater trochanter 32 is not necessary. For example, upon resection of natural head 28 and natural neck 30, the femur 14 may be reamed just below greater trochanter 32 so as to receive prosthesis 38 having recess 59 in place of attachment portion 54 as seen in Figure 5. Recess 59 may be filled with bone cement to secure the prosthesis 38 within the femur 14. Thus, the prosthesis 38 may be used to effectuate bone repair of hip joint 10 in situations where resection of greater trochanter 32 is not necessary.

Storing a separate implant to rectify each type of bone defect is undesirable as it is costly and requires a large amount of space. Thus, having one implant that is capable of rectifying numerous different defects is desirable. As discussed above, prosthesis 38 may be used not only when resection of greater trochanter 32 is necessary but also when resection is not necessary. Consequently, the use of prosthesis 38 is advantageous as it reduces inventory.

In order to restore the patient's natural range of motion, the trochanteric flange 56 must be secured to the surrounding soft tissue 36. As discussed above, the soft tissue attachment region 62 of the trochanteric flange 56 comprises one or more through holes 70 for receiving a suitable fastening device such as wire sutures 72, clamps (not shown), a suitable adhesive, or any other appropriate fastening device. The through holes 70 are threaded with, for example, the sutures 72 and the sutures 72 are secured to the surrounding soft tissue 36 so as to provide a connection between the trochanteric flange 56 and the surrounding soft tissue 36. As a result of this connection, the range of motion of the hip joint 10 with prosthesis 38 implanted is very similar to the range of motion experienced naturally.

The trochanteric flange 56 used with the prosthesis 38 may be selected from a group of trochanteric flanges 56a, 56b, or 56c, each flange 56 having a soft tissue attachment region 62 that varies in height as seen in Figures 6, 7, and 8. Each flange 56a, 56b, or 56c may be either integral or modular. The particular flange 56 selected for use with prosthesis 38 depends upon the amount of soft tissue 36 resected from the hip joint 10. If a large amount of soft tissue 36 is resected from the hip joint 10 then a trochanteric flange, such as flange 56a, having an extended soft tissue attachment region 62 is required (Figure 9). The use of a flange 56a having an elongated soft tissue attachment region 62 is necessary so as to allow for attachment of the trochanteric flange 56 to the surrounding soft tissue 36 without causing undesirable tension in the soft tissue 36. If a large amount of soft tissue 36 is resected and the remaining soft tissue 36 is sutured to a trochanteric flange having a shorter soft tissue attachment region 62, as found in flanges 56b or 56c (Figures 7 and 8), the soft tissue 36 must be stretched in order to engage the trochanteric flange 56. The tension created by stretching the soft tissue 36 is often so great that it causes the sutures to break and release the trochanter flange 56 from engagement with the soft tissue 36.

When little or no soft tissue 36 is resected from the hip joint 10 the use of a trochanteric flange, such as flange 56b or 56c, having a short soft tissue attachment region 62 is desirable (Figure 10). If little or no soft tissue 36 is resected from the hip joint 10 and a trochanteric flange 56 having an elongated soft tissue attachment region 62 is used, such as flange 56a, the attachment region 62 will protrude into the soft tissue 36 and cause damage or undesirable tension within the soft tissue 36. Consequently, it is desirable to use a trochanteric flange, such as flange 56b or 56c, having a shorter soft tissue attachment region 62.

If outfitted with a modular stem 42, the neck 44, head 50 and trochanteric flange 56 may be removed from the hip joint 10 even after the stem 42 has been secured within the intramedullary canal 40. The presence of modular stem 42 allows a physician to easily remove and repair the neck 44, head 50, and trochanteric flange 56 without having to remove the stem 42 from engagement with the intramedullary canal 40. Consequently, the prosthesis 38 may be easily repaired without resecting additional femur bone 14 as would be required to remove the secured stem 42 from the femur 14.

As an alternative to selecting flange 56 from a group of flanges 56a, 56b, or 56c, a single flange 56 that is adjustable in height, as seen in Figure 6 in phantom, may be used. For example, a flange 56 having a telescoping soft tissue attachment region 62 may be used to provide a single flange 56 capable of having any desirable height, such as a height approximating that of either flange 56a, 56b, or 56c. Further, a single flange 56 configured to receive soft tissue attachment regions 62 of various different heights may be used. The use of a single flange adjustable in height is desirable as it reduces inventory tracking and storage costs.

The use of modular stem 42 also aids in the trialing of the prosthesis 38. For example, if after the stem 42 has been secured within the intramedullary canal 40 it is discovered that the length or size of the neck 44, trochanteric flange 56, or head 50 is incorrect, the neck 44 may be removed and replaced with a neck 44, flange 56, or head 50 of the correct size. Thus, modular stem 42 allows for trialing of the prosthesis 38 even after the stem 42 has been secured within femur 14.

In use, the prosthesis 38 allows a physician to replace a resected proximal portion 24 of the femur 14 and the surrounding soft tissue 36. The prosthesis is generally comprised of a stem 42, a neck 44, a head 50, a trochanteric flange 56 and an acetabular cup 52. The stem, 42, neck 44, head 50, and flange 56 may be modular as seen in Figure 2, integral, or any combination of modular or integral components and may be of various different sizes according the particular application. The portion of the femur 14 remaining after resection is reamed and the stem 42 of prosthesis 38 is inserted within the reamed-out portion. The stem 42 is secured within the reamed-out portion by impaction or bone cement. The acetabular cup 52 is inserted within and secured to acetabulum 22 for receipt of head 50. If the greater trochanter 32 was resected, the trochanteric flange 56 is secured to the neck 44 and secured to the surrounding soft tissue 36 to restore the range of motion of the hip joint 10. If the greater trochanter 32 was not resected the prosthesis 38 may still be without the flange 56 as it is not required.

The trochanteric flange 56 may be modular or integral and is selected by the physician according to its size. If a large amount of soft tissue is resected the physician will select a trochanteric flange 56 with a large soft tissue attachment region 62. If a small amount of soft tissue is resected the physician will select trochanteric flange 56 with a small soft tissue attachment region 62. If after the prosthesis 38 is secured within the femur 14 it is discovered that the size of the neck 44, trochanteric flange 56, or head 50 is incorrect, the physician may remove the incorrect part and replace it with one of proper size without having to remove the stem 42 from the femur 14. Thus, the prosthesis 38 permits inter-operative trialing.

Thus, a femoral prosthesis 38 having a trochanteric flange 56 is provided. The trochanteric flange 56 may either be modular or integral. Additionally, all components of the prosthesis 38, such as stem 42, neck 44 and head 50 may be modular or integral and may be of various different sizes depending upon the particular application. The trochanteric flange 56 is selected from a group of trochanteric flanges, such as flanges 56a, 56b, and 56c, according to the amount of soft tissue 36 resected from the hip joint 10. For example, if a large amount of soft tissue 36 is resected a trochanteric flange, such as flange 56a, having an elongated soft tissue attachment region 62 is selected. If little or no soft tissue 36 is resected a trochanteric flange 56 having a shortened soft tissue attachment region 62, such as flange 56b or 56c, is selected. Selection of the proper trochanteric flange 56 allows the trochanteric flange 56 to closely approximate the surrounding soft tissue 36 and be secured to the soft tissue 36 without causing undesirable pressure or tension within the soft tissue 36, regardless of the amount of tissue resected. The prosthesis 38 may also be used without trochanteric flange 56 in situations where resection of the greater trochanter 32 is not necessary.

The description of the invention is merely exemplary in nature and, thus, variations that do not depart from the gist of the invention are intended to be within the scope of the invention. Such variations are not to be regarded as a departure from the spirit and scope of the invention.

## Claims

1. A hip prosthesis implantable in an intramedullary canal of a femur and secured to surrounding soft tissue, said hip prosthesis comprising:
a stem having a proximal end and a distal end, said distal end of said stem operable to be inserted into said intramedullary canal of said femur;
a neck extending from said proximal end of said stem;
a head extending from said neck, said head operable to articulate with at least one of an acetabular cup and a hip bone; and
a flange extending from said neck toward an ilium, said flange having a first attachment operable to secure said flange to said soft tissue, said flange being a first flange of a length depending upon the amount of soft tissue present.

2. The prosthesis of Claim 1, wherein said first flange is replaced by a second flange, said second flange extends to a greater distance from said neck than said first flange.

3. The prosthesis of Claim 1, wherein said first flange is adjustable in length so that said first flange may extend from said neck to a variety of different distances.

4. The prosthesis of Claim 1, wherein said first attachment is comprised of through holes extending through said flange selected from a group consisting of either a medial, a lateral, a longitudinal, an "X" shaped configuration, or any combination thereof.

5. The prosthesis of Claim 1, wherein either said stem, said neck, said head, or said flange is integral.

6. The prosthesis of Claim 1, wherein either said stem, said neck, said head, or said flange is modular.

7. The prosthesis of Claim 1, wherein said flange is secured to said neck using a second attachment means selected from a group consisting of a screw, a taper fit, a snap fit, a pin, an adhesive, a catch, a dovetail, a pinch, or any combination thereof.

8. The prosthesis of Claim 1, wherein said flange is secured to said surrounding soft tissue using a first attachment means selected from a group consisting of sutures, a clamp, an adhesive, or any combination thereof.

9. The prosthesis of Claim 1, wherein said flange is integral with said neck.

10. A hip prosthesis implantable in an intramedullary canal of a femur and secured to surrounding soft tissue, said hip prosthesis comprising:
a stem having a proximal end and a distal end, said distal end of said stem operable to be inserted into said intramedullary canal of said femur;
a neck extending from said proximal end of said stem;
a head extending from said neck, said head operable to articulate with at least one of an acetabular cup and a hip bone; and
a flange extending from said neck toward an ilium, said flange having a first attachment means for securing flange to said soft tissue and a second attachment means for securing flange to said neck, said flange being a first flange of length depending upon the amount of soft tissue present.

11. The prosthesis of Claim 10, wherein said first flange is replaced by a second flange, said second flange extends to a greater distance from said neck than said first flange.

12. The prosthesis of Claim 10, wherein said first flange is adjustable in length so that said first flange may extend from said neck to a variety of different distances.

13. The prosthesis of Claim 10, wherein said first attachment is comprised of through holes extending through said flange selected from a group consisting of either a medial, a lateral, a longitudinal, an "X" shaped configuration, or any combination thereof.

14. The prosthesis of Claim 10, wherein either said stem, said neck, said head, or said flange is integral.

15. The prosthesis of Claim 10, wherein either said stem, said neck, said head, or said flange is modular.

16. The prosthesis of Claim 10, wherein said flange is secured to said neck using a second attachment means selected from a group consisting of a screw, a taper fit, a snap fit, a pin, an adhesive, a catch, a dovetail, a pinch, or any combination thereof.

17. The prosthesis of Claim 10, wherein said flange is secured to said surrounding soft tissue using a first attachment means selected from a group consisting of sutures, a clamp, an adhesive, or any combination thereof.

18. A set of modular flanges with soft tissue attachment operable for replacing a greater trochanter comprising:
a first attachment region containing said soft tissue attachment; and
a second attachment region operable to secure said flanges to a hip prosthesis;
wherein a distance that said flanges extend from said hip prosthesis towards an illium differs among said flanges, said flanges being selected for their ability to approximate soft tissue surrounding a hip.

19. The modular flange of Claim 18, wherein said hip prosthesis is comprised of:
a stem having a proximal end and a distal end, said distal end of said stem operable to be inserted into an intramedullary canal of a femur;
a neck extending from said proximal end of said stem, said neck capable of receiving one of said flanges;
a head extending from said neck, said head operable to articulate with at least one of an acetabular cup and an acetabulum of a hip bone.

20. The prosthesis of Claim 19, wherein said first attachment is comprised of through holes extending through said flange selected from a group consisting of either a medial, a lateral, a longitudinal, or an "X" shaped configuration, or any combination thereof.

21. The prosthesis of Claim 19, wherein either said stem, said neck, said head, or said flange is integral.

22. The prosthesis of Claim 19, wherein either said stem, said neck, said head, or said flange is modular.

23. The prosthesis of Claim 19, wherein said flange is secured to said neck using a second attachment means selected from a group consisting of a screw, a taper fit, a snap fit, a pin, an adhesive, a catch, a dovetail, a pinch, or any combination thereof.

24. The prosthesis of Claim 19, wherein said flange is secured to said surrounding soft tissue using a first attachment means selected from a group consisting of sutures, a clamp, an adhesive, or any combination thereof.

25. A method for replacing resected portions of a proximal femur comprising:
providing a prosthesis having a stem for insertion within a femoral canal, a head for articulation with an acetabular cup, and a neck for supporting a greater trochanter implant on said prosthesis;
selecting said greater trochanter implant from a group of flanges, each of said flanges differing in size;
attaching said selected flange to said neck;
placing said stem within a reamed out portion of said femur;
inserting said head portion within said acetabulum;
securing said flange to soft tissue surrounding said femur using a suitable fastening device.

26. The method of Claim 25, further comprising attaching said flange to soft tissue by passing a suture through the soft tissue and through holes extending through said flange selected from a group consisting of either a medial, a lateral, a longitudinal, an "X" shaped configuration, or any combination thereof.

27. The method of Claim 25, wherein attaching said flange includes attaching said flange to said neck using an attachment mechanism selected from a group consisting of a screw, a taper fit, a snap fit, a pin, an adhesive, a catch, a dovetail, a pinch, or any combination thereof.
